# EUROPEAN PATENT SPECIFICATION

(11) **EP 4 504 182 B1**
(45) Date of publication and mention of the grant of the patent: **25.11.2026**
(21) Application number: 23719651.4
(22) Date of filing: 06.04.2023
(51) Int. Cl.: A61K 31/4166, A61K 45/06, A61K 31/496, A61P 35/00, A61P 35/04

(54) **COMBINATION THERAPY FOR TREATING CANCER**
KOMBINATIONSTHERAPIE ZUR BEHANDLUNG VON KREBS
POLYTHÉRAPIE POUR LE TRAITEMENT DU CANCER

(30) Priority: 07.04.2022 US 202263362613 P
(43) Date of publication of application: 12.02.2025
(73) Proprietor: Astrazeneca AB, 151 85 Södertälje (SE)
(72) Inventor: COSULICH, Sabina Chiara, Cambridge Cambridgeshire CB2 0AA (GB); ALBERTELLA, Mark R, Cambridge Cambridgeshire CB2 0AA (GB); BROWN, Jessica S, Cambridge Cambridgeshire CB2 0AA (GB); LEO, Elisabetta, Cambridge Cambridgeshire CB2 0AA (GB)
(74) Representative: AstraZeneca Intellectual Property
(86) International application number: PCT/EP2023/059130
(87) International publication number: WO 2023/194530

(56) References cited:
- LI LIKUN ET AL: "Androgen receptor inhibitor-induced "BRCAness" and PARP inhibition are synthetically lethal for castration-resistant prostate cancer", SCIENCE SIGNALING, no. 10, 23 May 2017 (2017-05-23), pages 1 - 11, XP093055940
- JOHANNES JEFFREY W. ET AL: "Discovery of 5-{4-[(7-Ethyl-6-oxo-5,6-dihydro-1,5-naphthyridin-3-yl)methyl]piperazin-1-yl}- N -methylpyridine-2-carboxamide (AZD5305): A PARP1-DNA Trapper with High Selectivity for PARP1 over PARP2 and Other PARPs", JOURNAL OF MEDICINAL CHEMISTRY, vol. 64, no. 19, 27 September 2021 (2021-09-27), US, pages 14498 - 14512, XP093028792, ISSN: 0022-2623, Retrieved from the Internet <URL:https://pubs.acs.org/doi/pdf/10.1021/acs.jmedchem.1c01012> DOI: 10.1021/acs.jmedchem.1c01012
- AGARWAL NEERAJ ET AL: "Talazoparib plus enzalutamide in metastatic castration-resistant prostate cancer: TALAPRO-2 phase III study design", FUTURE ONCOLOGY, vol. 18, no. 4, 26 January 2022 (2022-01-26), GB, pages 425 - 436, XP093056092, ISSN: 1479-6694, DOI: 10.2217/fon-2021-0811
- STANISZEWSKA A D ET AL: "The novel PARP1-selective inhibitor, AZD5305, is efficacious as monotherapy and in combination with standard of care chemotherapy in the in vivo preclinical models", CANCER RESEARCH, AMERICAN ASSOCIATION FOR CANCER RESEARCH, US, vol. 81, no. 13 SUPPL, 1 July 2021 (2021-07-01), XP009532404, ISSN: 1538-7445, DOI: 10.1158/1538-7445.AM2021-1270
- ANONYMOUS: "Study of AZD5305 When Given in Combination With New Hormonal Agents in Patients With Metastatic Prostate Cancer - Full Text View - ClinicalTrials.gov", 10 May 2022 (2022-05-10), XP093055809, Retrieved from the Internet <URL:https://www.clinicaltrials.gov/ct2/show/NCT05367440>
- AGARWAL NEERAJ ET AL: "Talazoparib plus enzalutamide in men with first-line metastatic castration-resistant prostate cancer (TALAPRO-2): a randomised, placebo-controlled, phase 3 trial", THE LANCET, 1 June 2023 (2023-06-01), AMSTERDAM, NL, XP093055981, ISSN: 0140-6736, DOI: 10.1016/S0140-6736(23)01055-3

## Description

The present disclosure relates to methods of treating metastatic prostate cancer, hormone sensitive prostate cancer (HSPC) and castrate resistant prostate cancer (CRPC) in a patient in need thereof.

### Background

Prostate cancer is the second most common cancer in men. With an estimated 375,304 deaths in 2020 worldwide, prostate cancer is the fifth leading cause of death from cancer in men and represents 6.8% of total cancer death in males (Sung 2021).

Treatment of prostate cancer with androgen deprivation therapy (ADT) such as luteinising hormone-releasing hormone (LHRH) analogues or orchidectomy is usually initially effective at controlling metastatic disease. However, patients inevitably progress from an androgen sensitive to a castration-resistant phenotype which is associated with 90% of overall mortality (Scher 2015).

The recent approval of several new hormonal agents (NHAs) has significantly altered the treatment landscape for patients with metastatic castrate resistant prostate cancer (mCRPC) and NHAs are now considered standard of care in both the mCRPC and metastatic hormone sensitive prostate cancer (MHSPC) settings (Mohler 2019, Parker 2020).

Both abiraterone acetate and enzalutamide in combination with ADT have demonstrated robust improvements in progression free survival (PFS) and overall survival (OS) and have shown a significantly prolonged time to initiation of cytotoxic chemotherapy in patients with CRPC (Beer 2014, Ryan 2013).

Additionally, recent data have demonstrated the benefit of NHAs in patients with mHSPC. Abiraterone acetate plus prednisone with ADT demonstrated significant survival benefits compared with ADT alone, by further prolonging OS and delaying initiation of chemotherapy and subsequent therapy (Fizazi 2019). Enzalutamide plus ADT significantly reduced the risk of radiographic progression or death versus placebo plus ADT as well as reduced risk of PSA progression, initiation of new antineoplastic therapy, first symptomatic skeletal event, castration resistance, and pain progression (Armstrong 2019)

A Phase III trial is ongoing to evaluate darolutamide in combination with standard ADT in patients with mHSPC (ARANOTE, NCT04736199).

The addition of Olaparib (a PARP1/PARP2 inhibitor) to abiraterone acetate plus ADT has demonstrated an improvement in radiographic progression-free survival (rPFS) compared with abiraterone acetate alone for both men with mCRPC who had previously received docetaxel (Clarke 2018), and those who had not received a prior line of systemic therapy, irrespective of homologous recombination repair gene mutation (HRRm) status (AstraZeneca Press Release 24 September 2021).

It is not expected that Olaparib (a PARP1/PARP2 inhibitor) could be successfully used in combination with enzalutamide as enzalutamide is a strong CYP3A4 inducer (Gibbons 2015) and Olaparib is a substrate of CYP3A4 (Dirix 2016), so co-administration of enzalutamide with Olaparib in a multiple dose setting would significantly reduce Olaparib exposure in patients. Likun Li et al. report a treatment strategy, in which enzalutamide was followed by the PARP inhibitor olaparib, promoted DNA damage-induced cell death and inhibited clonal proliferation of prostate cancer cells in culture and suppressed the growth of prostate cancer xenografts in mice (Li, Likun, et al. "Androgen receptor inhibitor- induced "BRCAness" and PARP inhibition are synthetically lethal for castration-resistant prostate cancer." Science signaling 10.480 (2017): eaam7479).

While much progress has been made in the treatment of metastatic prostate cancer, hormone sensitive prostate cancer (HSPC) and castrate resistant prostate cancer (CRPC), including metastatic hormone sensitive prostate cancer (mHSPC) and metastatic castrate resistant prostate cancer (mCRPC), many patients who have such cancers live with an incurable disease. Accordingly, it is important to continue to find new treatments for patients with incurable cancer.

### Summary

The invention is set out in the appended set of claims.

The references to methods of treatment by therapy of this description are to be interpreted as references to compounds, pharmaceutical compositions and medicaments of the present invention for use in those methods.

In some embodiments, disclosed is a method of treating metastatic prostate cancer, hormone sensitive prostate cancer (HSPC) or castrate resistant prostate cancer (CRPC) in a subject in need thereof, comprising administering to the subject a first amount of AZD5305 or a pharmaceutically acceptable salt thereof, and a second amount of enzalutamide or a pharmaceutically acceptable salt thereof. In the method, the first amount and the second amount together comprise a therapeutically effective amount.

In some embodiments, disclosed is AZD5305, or a pharmaceutically acceptable salt thereof, for use in the treatment of metastatic prostate cancer, hormone sensitive prostate cancer (HSPC) or castrate resistant prostate cancer (CRPC) in a subject, wherein said treatment comprises the separate, sequential or simultaneous administration of i) said AZD5305, or a pharmaceutically acceptable salt thereof, and ii) enzalutamide, or a pharmaceutically acceptable salt thereof, to said subject.

In some embodiments, disclosed is enzalutamide, or a pharmaceutically acceptable salt thereof, for use in the treatment of metastatic prostate cancer, hormone sensitive prostate cancer (HSPC) or castrate resistant prostate cancer (CRPC) in a subject, wherein said treatment comprises the separate, sequential or simultaneous administration of i) said enzalutamide, or a pharmaceutically acceptable salt thereof, and ii) AZD5305, or a pharmaceutically acceptable salt thereof, to said subject.

In some embodiments, disclosed is the use of AZD5305, or a pharmaceutically acceptable salt thereof, in the manufacture of a medicament for use in the treatment of metastatic prostate cancer, hormone sensitive prostate cancer (HSPC) or castrate resistant prostate cancer (CRPC), wherein said treatment comprises the separate, sequential or simultaneous administration of i) said medicament comprising AZD5305, or a pharmaceutically acceptable salt thereof, and ii) enzalutamide, or a pharmaceutically acceptable salt thereof, to said subject.

In the above embodiments, the metastatic prostate cancer may be metastatic hormone sensitive prostate cancer (mHSPC) or metastatic castrate resistant prostate cancer (mCRPC).

In some embodiments, disclosed is a pharmaceutical product comprising i) AZD5305 or a pharmaceutically acceptable salt thereof, and ii) enzalutamide or a pharmaceutically acceptable salt thereof.

In some embodiments, disclosed is a kit comprising: a first pharmaceutical composition comprising AZD5305, or a pharmaceutically acceptable salt thereof; a second pharmaceutical composition comprising enzalutamide, or a pharmaceutically acceptable salt thereof; and instructions for using the first and second pharmaceutical compositions in combination.

The combination of AZD5305 and enzalutamide may result in fewer side effects or be more effective than current monotherapies or combination therapies. This may result from AZD5305 being a selective PARP1 inhibitor. By 'selective PARP1 inhibitor' it is meant an inhibitor of the PARP enzyme having greater selectivity for PARP1 over other members of the PARP family, such as PARP2, PARP3, PARP5a, and PARP6. In some embodiments the selective PARP1 inhibitor has a selectivity for PARP1 over PARP2. In some embodiments, the selective PARP1 inhibitor has a selectivity for PARP1 over PARP2 which is greater than 5:1. In some embodiments, the selective PARP1 inhibitor has a selectivity for PARP1 over PARP2 which is greater than 10:1. In some embodiments, the selective PARP1 inhibitor has a selectivity for PARP1 over PARP2 which is greater than 100:1.

In some embodiments, disclosed is a method of treating metastatic prostate cancer, hormone sensitive prostate cancer (HSPC) or castrate resistant prostate cancer (CRPC) in a subject in need thereof, comprising administering to the subject a first amount of a selective PARP1 inhibitor, namely AZD5305, or a pharmaceutically acceptable salt thereof, and a second amount of enzalutamide or a pharmaceutically acceptable salt thereof. In the method, the first amount and the second amount together comprise a therapeutically effective amount.

In some embodiments, disclosed is AZD5305 or a pharmaceutically acceptable salt thereof, for use in the treatment of metastatic prostate cancer, hormone sensitive prostate cancer (HSPC) or castrate resistant prostate cancer (CRPC) in a subject, wherein said treatment comprises the separate, sequential or simultaneous administration of i) said AZD5305 or a pharmaceutically acceptable salt thereof, and ii) enzalutamide, or a pharmaceutically acceptable salt thereof, to said subject.

In some embodiments, disclosed is enzalutamide, or a pharmaceutically acceptable salt thereof, for use in the treatment of metastatic prostate cancer, hormone sensitive prostate cancer (HSPC) or castrate resistant prostate cancer (CRPC) in a subject, wherein said treatment comprises the separate, sequential or simultaneous administration of i) said enzalutamide, or a pharmaceutically acceptable salt thereof, and ii) AZD5305 or a pharmaceutically acceptable salt thereof, to said subject.

### Brief Description of the Drawings

Figure 1 illustrates the efficacy and tolerability of AZD5305 combined with enzalutamide in an *in vivo* pre-clinical model LNCaP
Figure 2 illustrates the efficacy of AZD5305 combined with enzalutamide in an *in vivo* pre-clinical model C901

### Detailed Description

The term "AZD5305" refers to a compound with the chemical name 5-{4-[(7-ethyl-6-oxo-5,6-dihydro-1,5-naphthyridin-3-yl)methyl]piperazin-1-yl}-N-methylpyridine-2-carboxamide and structure shown below:

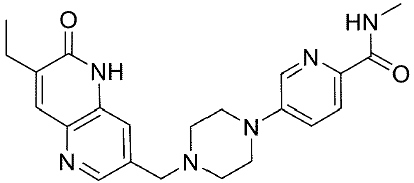

AZD5305 is a potent and selective PARP1 inhibitor and PARP1-DNA trapper with excellent *in vivo* efficacy. AZD5305 is highly selective for PARP1 over other PARP family members, with good secondary pharmacology and physicochemical properties and excellent pharmacokinetics in preclinical species, and with reduced effects on human bone marrow progenitor cells *in vitro.*

The synthesis of AZD5305 is described in Johannes 2021 and in WO2021/013735.

In some embodiments, a free base AZD5305 is administered to a subject. In some embodiments, a pharmaceutically acceptable salt of AZD5305 is administered to a subject. In some embodiments, crystalline AZD5305 or a pharmaceutically acceptable salt of AZD5305 is administered to a subject.

The term "enzalutamide" refers to a compound with the chemical name 4-(3-(4-cyano-3-(trifluoromethyl)phenyl)-5,5-dimethyl-4-oxo-2-thioxoimidazolidin-1-yl)-2-fluoro-N-methylbenzamide and structure shown below:

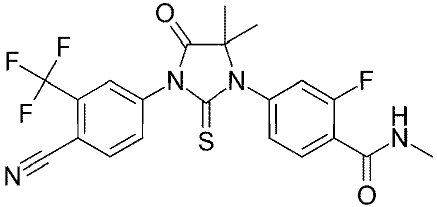

Enzalutamide is a potent AR (Androgen Receptor) signaling inhibitor that blocks several steps in the AR signaling pathway. Enzalutamide competitively inhibits binding of androgens to AR, inhibits nuclear translocation of activated receptors and inhibits the association of the activated AR with DNA even in the setting of AR over-expression and in prostate cancer cells resistant to antiandrogens. Enzalutamide treatment decreases the growth of prostate cancer cells and can induce cancer cell death and tumour regression. Enzalutamide is indicated for the treatment of adult men with mCRPC who are asymptomatic or mildly symptomatic after failure of ADT in whom chemotherapy is not yet clinically indicated, and for the treatment of patients with mCSPC.

The synthesis of enzalutamide is described in US7709517.

In some embodiments, a free base enzalutamide is administered to a subject. In some embodiments, a pharmaceutically acceptable salt of enzalutamide is administered to a subject.

The language "pharmaceutical composition" includes compositions comprising an active ingredient and a pharmaceutically acceptable excipient, carrier or diluent, wherein the active ingredient is AZD5305 or a pharmaceutically acceptable salt thereof, or enzalutamide or a pharmaceutically acceptable salt thereof. The language "pharmaceutically acceptable excipient, carrier or diluent" includes compounds, materials, compositions, and/or dosage forms which are, within the scope of sound medical judgment, suitable for use in contact with the tissues of human beings and animals without excessive toxicity, irritation, allergic response, or other problem or complication, as ascertained by one of skill in the art. In some embodiments, the pharmaceutical compositions are in solid dosage forms, such as capsules, tablets, granules, powders or sachets. In some embodiments, the pharmaceutical compositions are in the form of a sterile injectable solution in one or more aqueous or non-aqueous non-toxic parenterally acceptable buffer systems, diluents, solubilizing agents, cosolvents, or carriers. A sterile injectable preparation may also be a sterile injectable aqueous or oily suspension or suspension in a non-aqueous diluent, carrier or co-solvent, which may be formulated according to known procedures using one or more of the appropriate dispersing or wetting agents and suspending agents. The pharmaceutical compositions could be a solution for iv bolus/infusion injection or a lyophilized system (either alone or with excipients) for reconstitution with a buffer system with or without other excipients. The lyophilized freeze-dried material may be prepared from non-aqueous solvents or aqueous solvents. The dosage form could also be a concentrate for further dilution for subsequent infusion.

The language "treat," "treating" and "treatment" includes the reduction or inhibition of enzyme or protein activity related to PARP-1, AR or metastatic prostate cancer, hormone sensitive prostate cancer (HSPC) or castrate resistant prostate cancer (CRPC) in a subject, amelioration of one or more symptoms of metastatic prostate cancer, hormone sensitive prostate cancer (HSPC) or castrate resistant prostate cancer (CRPC) in a subject, or the slowing or delaying of progression of metastatic prostate cancer, hormone sensitive prostate cancer (HSPC) or castrate resistant prostate cancer (CRPC) in a subject. The language "treat," "treating" and "treatment" also includes the reduction or inhibition of the growth of a tumor or proliferation of cancerous cells in a subject.

The language "inhibit", "inhibition" or "inhibiting" includes a decrease in the baseline activity of a biological activity or process.

The term "subject" includes warm-blooded mammals, for example, primates, dogs, cats, rabbits, rats, and mice. In some embodiments, the subject is a primate, for example, a human. In some embodiments, the subject is suffering from metastatic prostate cancer, hormone sensitive prostate cancer (HSPC) or castrate resistant prostate cancer (CRPC).

The language "therapeutically effective amount" includes that amount of AZD5305 and that amount of enzalutamide which together will elicit a biological or medical response in a subject, for example, the reduction or inhibition of enzyme or protein activity related to PARP1, AR, or cancer; amelioration of symptoms of metastatic prostate cancer, hormone sensitive prostate cancer (HSPC) or castrate resistant prostate cancer (CRPC); or the slowing or delaying of progression of metastatic prostate cancer, hormone sensitive prostate cancer (HSPC) or castrate resistant prostate cancer (CRPC). In some embodiments, the language "therapeutically effective amount" includes the amount of AZD5305 and enzalutamide together that is effective to at least partially alleviate, inhibit, and/or ameliorate metastatic prostate cancer, hormone sensitive prostate cancer (HSPC) or castrate resistant prostate cancer (CRPC) or inhibit PARP1 or AR, and/or reduce or inhibit the growth of a tumor or proliferation of cancerous cells in a subject.

In some embodiments, disclosed is a method of treating metastatic prostate cancer, hormone sensitive prostate cancer (HSPC) or castrate resistant prostate cancer (CRPC) in a subject in need thereof, comprising administering to the subject a first amount of AZD5305 or a pharmaceutically acceptable salt thereof, and a second amount of enzalutamide or a pharmaceutically acceptable salt thereof. In the method, the first amount and the second amount together comprise a therapeutically effective amount.

In some embodiments, disclosed is AZD5305, or a pharmaceutically acceptable salt thereof, for use in the treatment of metastatic prostate cancer, hormone sensitive prostate cancer (HSPC) or castrate resistant prostate cancer (CRPC) in a subject, wherein said treatment comprises the separate, sequential or simultaneous administration of i) said AZD5305, or a pharmaceutically acceptable salt thereof, and ii) enzalutamide, or a pharmaceutically acceptable salt thereof, to said subject.

In some embodiments, disclosed is enzalutamide, or a pharmaceutically acceptable salt thereof, for use in the treatment of metastatic prostate cancer, hormone sensitive prostate cancer (HSPC) or castrate resistant prostate cancer (CRPC) in a subject, wherein said treatment comprises the separate, sequential or simultaneous administration of i) said enzalutamide, or a pharmaceutically acceptable salt thereof, and ii) AZD5305, or a pharmaceutically acceptable salt thereof, to said subject.

In some embodiments, disclosed is the use of AZD5305, or a pharmaceutically acceptable salt thereof, in the manufacture of a medicament for use in the treatment of metastatic prostate cancer, hormone sensitive prostate cancer (HSPC) or castrate resistant prostate cancer (CRPC) in a subject, wherein said treatment comprises the separate, sequential or simultaneous administration of i) said medicament comprising AZD5305, or a pharmaceutically acceptable salt thereof, and ii) enzalutamide, or a pharmaceutically acceptable salt thereof, to said subject.

In some embodiments, AZD5305 or a pharmaceutically acceptable salt thereof and enzalutamide or a pharmaceutically acceptable salt thereof are administered separately, sequentially or simultaneously in a treatment cycle. In some embodiments, AZD5305 or a pharmaceutically acceptable salt thereof is continuously administered in the treatment cycle and enzalutamide or a pharmaceutically acceptable salt is also continuously administered in the treatment cycle.

The term "continuous" or "continuously" refers to administration of a therapeutic agent, e.g. AZD5305, at regular intervals without stopping or interruption, i.e., no void day. By "void day", it is meant a day when a therapeutic agent is not administered.

A "cycle", "treatment cycle" or "dosing schedule", as used herein, refers to a period of combination treatment that is repeated on a regular schedule. For example, the treatment can be given for one week, two weeks, or three weeks wherein AZD5305 and enzalutamide are administered in a coordinated fashion. In some embodiments, a treatment cycle is about 1 week to about 3 months. In some embodiments, a treatment cycle is about 5 days to about 1 month. In some embodiments, a treatment cycle is about 1 week to about 3 weeks. In some embodiments, a treatment cycle is about 1 week, about 10 days, about 2 weeks, about 3 weeks, about 4 weeks, about 2 months, or about 3 months.

In some embodiments, AZD5305 or a pharmaceutically acceptable salt thereof and enzalutamide or a pharmaceutically acceptable salt thereof are administered to the human subject in one or more treatment cycles, e.g., a treatment course. A "treatment course" comprises multiple treatment cycles, which can be repeated on a regular schedule, or adjusted as a tapered schedule as the patient's disease progression is monitored. For example, a patient's treatment cycles can have longer periods of treatment and/or shorter periods of rest at the beginning of a treatment course (e.g., when the patient is first diagnosed), and as the cancer enters remission, the rest period lengthens, thereby increasing the length of one treatment cycle. The period of time for treatment and rest in a treatment cycle, the number of treatment cycles, and the length of time for the treatment course can be determined and adjusted throughout the treatment course by the skilled artisan based on the patient's disease progression, treatment tolerance, and prognosis. In some embodiments, the method comprises 1 to 10 treatment cycles. In some embodiments, the method comprises 2 to 8 treatment cycles.

In some embodiments, AZD5305 or a pharmaceutically acceptable salt thereof is administered for 7 days in a 7-day pre-treatment cycle, and then 28 days in a 28-day treatment cycle, and enzalutamide or a pharmaceutically acceptable salt thereof is administered for 28 days in the 28-day treatment cycle.

In some embodiments, AZD5305 or a pharmaceutically acceptable salt thereof is administered orally. In some embodiments, AZD5305 or a pharmaceutically acceptable salt thereof is in tablet dosage form. In some embodiments, AZD5305 is administered in a dose of up to about 60 mg (for example, up to about 5 mg, up to about 10 mg, up to about 15 mg, up to about 20 mg, up to about 25 mg, up to about 30 mg, up to about 35 mg, up to about 40 mg, up to about 45 mg, up to about 50 mg, up to about 55 mg, or up to about 60 mg AZD5305) per day. In some embodiments, AZD5305 is administered once a day (QD). In some embodiments, AZD5305 is administered in a dose of about 10 mg QD, about 15 mg QD, about 20 mg QD, about 25 mg QD, about 30 mg QD, about 35 mg QD, about 40 mg QD, about 45 mg QD, about 50 mg QD, about 55 mg QD or about 60 mg QD.

In some further embodiments, AZD5305 is administered in a dose of up to about 140 mg (for example, up to about 80 mg, up to about 90 mg, up to about 100 mg, up to about 110 mg, up to about 120 mg, or up to about 140 mg AZD5305) per day. In some further embodiments, AZD5305 is administered in a dose of about 80 mg QD, about 90 mg QD, about 100 mg QD, about 110 mg QD, about 120 mg QD, or about 140 mg QD.

In some embodiments, enzalutamide or a pharmaceutically acceptable salt thereof is administered orally. In some embodiments, enzalutamide or a pharmaceutically acceptable salt thereof is in tablet dosage form. In some embodiments, enzalutamide or a pharmaceutically acceptable salt thereof is in capsule dosage form. In some embodiments, enzalutamide or a pharmaceutically acceptable salt thereof is administered in a dose of about 160 mg orally once a day (QD). In some embodiments, the 160 mg dose comprises four 40mg capsules, four 40mg tablets or two 80 mg tablets.

In some embodiments, AZD5305 and enzalutamide are taken together on an empty stomach, with no food two hours before, and one hour after.

In some embodiments, disclosed is a pharmaceutical product comprising i) AZD5305 or a pharmaceutically acceptable salt thereof, and ii) enzalutamide or a pharmaceutically acceptable salt thereof. In some embodiments, AZD5305 or a pharmaceutically acceptable salt thereof, and enzalutamide or a pharmaceutically acceptable salt thereof are present in a single dosage form. In some embodiments, AZD5305 or a pharmaceutically acceptable salt thereof, and enzalutamide or a pharmaceutically acceptable salt thereof are present separate dosage forms.

In some embodiments, disclosed is a kit comprising: a first pharmaceutical composition comprising AZD5305, or a pharmaceutically acceptable salt thereof; a second pharmaceutical composition comprising enzalutamide, or a pharmaceutically acceptable salt thereof; and instructions for using the first and second pharmaceutical compositions in combination.

Metastatic prostate cancer refers to prostate cancer which has spread or metasised to another part of the body.

Hormone sensitive prostate cancer (HSPC) refers to prostate cancer whose growth is inhibited by a decrease in androgen levels or by inhibiting androgen action.

Castrate resistant prostate cancer (CRPC) refers to prostate cancer which continues to grow even when androgen levels in the body are extremely low or undetectable.

Metastatic hormone sensitive prostate cancer (mHSPC) refers to prostate cancer which has spread or metasised to another part of the body, and whose growth is inhibited by a decrease in androgen levels or by inhibiting androgen action.

Metastatic castrate resistant prostate cancer (mCRPC) refers to prostate cancer which has spread or metasised to another part of the body, and which continues to grow even when androgen levels in the body are extremely low or undetectable.

In some embodiments, treatment with a luteinising hormone-releasing hormone (LHRH) agonist or antagonist may be administered concurrently, especially if the patient has not undergone an orchidectomy or a subcapsular orchidectomy. LHRH agonists include leuprolide/leuprorelin, goserelin, triptorelin, histrelin, and buserelin. LHRH antagonists include degarelix, relugolix, bicalutamide, flutamide and cyproterone acetate. Such additional treatments may be dosed at the current standard of care.

Without wishing to be bound by theory, the combination of AZD5305 and enzalutamide may be beneficial as PARP1 is a positive co-regulator of the AR-driven gene expression of AR targets, in addition to its role in DNA repair. (Schiewer 2012; Schiewer and Knudsen 2014). As a result, AZD5305 should further inactivate the androgen receptor pathway, adding to the effect of enzalutamide.

In addition, New Hormonal Agents (NHAs) have been shown to induce an HRR-deficient phenotype through inhibition of AR signalling (Asim 2017; Goodwin 2013; Li 2017; Polkinghorn 2013; Tarish 2015). Homologous recombination repair gene transcripts and protein levels were found to be upregulated in response to enhanced AR signalling in prostate cancer, and increased radioresistance was observed in the presence of functional AR signalling while decreased HRR gene expression was seen in NHA-treated cells and tumour biopsies. As a result, without wishing to be bound by theory, the induction of an HRR-deficient phenotype by a NHA will lead to increased sensitivity to AZD5305, a selective PARP-1 inhibitor.

In some embodiments, the prostate cancer treated may be deficient in Homologous Recombination (HR) dependent DNA DSB repair activity. The HR dependent DNA DSB repair pathway repairs double-strand breaks (DSBs) in DNA via homologous mechanisms to reform a continuous DNA helix (Khanna and Jackson 2001). The components of the HR dependent DNA DSB repair pathway include, but are not limited to, ATM (NM_000051), RAD51 (NM_002875), RAD51L1 (NM_002877), RAD51C (NM_002876), RAD51L3 (NM_002878), DMC1 (NM_007068), XRCC2 (NM_005431), XRCC3 (NM_005432), RAD52 (NM_002879), RAD54L (NM_003579), RAD54B (NM_012415), BRCA1 (NM_007295), BRCA2 (NM_000059), RAD50 (NM_005732), MRE11A (NM_005590) and NBS1 (NM_002485). Other proteins involved in the HR dependent DNA DSB repair pathway include regulatory factors such as EMSY (Hughes-Davies 2003). HR components are also described in Wood 2001.

A prostate cancer which is deficient in HR dependent DNA DSB repair may comprise or consist of one or more cancer cells which have a reduced or abrogated ability to repair DNA DSBs through that pathway, relative to normal cells i.e. the activity of the HR dependent DNA DSB repair pathway may be reduced or abolished in the one or more cancer cells.

The activity of one or more components of the HR dependent DNA DSB repair pathway may be abolished in the one or more prostate cancer cells of an individual having a prostate cancer which is deficient in HR dependent DNA DSB repair. Components of the HR dependent DNA DSB repair pathway are well characterised in the art (see for example, Wood 2001) and include the components listed above.

In some embodiments, the prostate cancer cells may have a BRCA1 and/or a BRCA2 deficient phenotype i.e. BRCA1 and/or BRCA2 activity is reduced or abolished in the prostate cancer cells. Prostate cancer cells with this phenotype may be deficient in BRCA1 and/or BRCA2, i.e. expression and/or activity of BRCA1 and/or BRCA2 may be reduced or abolished in the prostate cancer cells, for example by means of mutation or polymorphism in the encoding nucleic acid, or by means of amplification, mutation or polymorphism in a gene encoding a regulatory factor, for example the EMSY gene which encodes a BRCA2 regulatory factor (Hughes-Davies 2003).

BRCA1 and BRCA2 are known tumour suppressors whose wild-type alleles are frequently lost in tumours of heterozygous carriers (Jasin 2002; Tutt 2002).

In some embodiments, the individual is heterozygous for one or more variations, such as mutations and polymorphisms, in BRCA1 and/or BRCA2 or a regulator thereof. The detection of variation in BRCA1 and BRCA2 is well-known in the art and is described, for example in EP 699 754, EP 705 903, Neuhausen and Ostrander 1992; Chappuis and Foulkes 2002; Janatová 2003; Jancárková 2003). Determination of amplification of the BRCA2 binding factor EMSY is described in Hughes-Davies 2003.

Mutations and polymorphisms associated with cancer may be detected at the nucleic acid level by detecting the presence of a variant nucleic acid sequence or at the protein level by detecting the presence of a variant (i.e. a mutant or allelic variant) polypeptide.

### Examples

The compounds of the application will now be further explained by reference to the following non-limiting examples.

### Example 1. Efficacy of AZD5305 combined with enzalutamide in an in vitro assay

### Cell lines

The following cell lines were originally obtained from ATCC:

| **Cell Lines** | **Source** | **AR status** | **AR variants** | **Reference** |
|---|---|---|---|---|
| LnCAP | mHSPC | AR+, T877A | AR-FL | Cunningham and You 2015 |
| C4-2 | mCRPC | AR+, ARV7, AR8 | AR-FL, ARV7 | Cunningham and You 2015 |
| VCAP | mHSPC | AR+ | AR-FL, ARV7 | Cunningham and You 2015 |
| CWR22Pc-R1-AD1 | mCRPC | AR+ | AR-FL, ARV8 | Nyquist 2013 |

Cell line identification was validated using the CellCheck assay (IDEXX Bioanalytics, Westbrook, ME, USA). All cell lines were validated free of virus Mycoplasma contamination using the MycoSEQ assay (Thermo Fisher Scientific, Waltham, MA, USA) or STAT-Myco assay (IDEXX Bioanalytics). All cell lines were grown RPMI-1640 growth media (Corning 17-105-CV) supplemented with 10% fetal bovine serum (FBS) or, when indicated, 10% charcoal stripped FBS (ThermoFisher Scientific, 12676029) and 2 mM glutamine.

### Cell proliferation assay and combination benefit calculation

Cells in 384-well or 96-well plates were dosed using an Echo 555 (LabCyte, San Jose, CA, USA) or using the HP D300e Digital Dispenser (HP Life Science Dispensing), respectively. Live cell count pre- and post-treatment (7 days after treatment) was determined using CellTiter-Glo as per manufacturer's instructions (Promega, Madison, WI, USA; G7570).

Cell viability was determined with the Sytox Green assay as described in Davies 2012 and the AC₅₀ calculated. The HSA (Highest Single Agent) Synergy Score was calculated according to Bernenbaum 1989.

### Results

| **Cell Line** | **AZD5305, M** | | **Enzalutamide, M** | | **Synergy score** | |
|---|---|---|---|---|---|---|
| | **AC₅₀** | **±SD** | **AC₅₀** | **±SD** | **HSA** | **±SD** |
| **LnCAP** | 1.2E-05 | 8.32E-08 | 1.41E-06 | 1.37E-06 | 0.9098 | 0.9989 |
| **C4-2** | 0.00002 | 0 | 8.33E-06 | 2.89E-06 | 0.77 | 0.3395 |
| **VCAP** | **1.6E-06** | 2.8E-06 | 2.94E-06 | 6.51E-07 | 1.433 | 0.05317 |
| CWR22Pc-R1-AD1 | 3.16E-08 | 1.85E-08 | 9.59E-06 | 7.1E-07 | 2.611 | 1.719 |

Monotherapy agent's potency is expressed in M concentrations; values are the mean of two independent experiments each performed in triplicate. HSA (Highest Single Agent) is the synergy score mean of three independent experiments each performed in triplicate. SD indicate standard deviation error, where not indicated only one experiment was performed.

These results show that the combination of AZD5305 and enzalutamide showed synergy in the LnCAP, C4-2, VCAP and CWR22Pc-R1-AD1 cell lines.

### Example 2. Efficacy of AZD5305 combined with enzalutamide in an in vitro assay in ATM KO prostate cancer cells

An isogenic ATM-KO model in LNCAP cells was generated with CRISPR-Cas9 technology. Two clones were obtained that displayed very low or undetectable ATM protein levels, clone.1 (0.14 ATM protein level compared to the control) and clone.2 (<0.01 ATM protein level compared to the control).

Using the method of example 1, these ATM-KO cell lines were determined to be sensitive to AZD5305, with an AC₅₀ of 3 nM, in contrast to the parental (or control) LNCAP cell line which is not sensitive to AZD5305 monotherapy, with an AC₅₀ greater than 10 µM. No sensitivity could be measured in the cell lines in response to enzalutamide.

| | **AZD5305, AC₅₀ (µM)** | **Enzalutamide, AC₅₀ (µM)** |
|---|---|---|
| **LnCAP parental** | >10 | >10 |
| **LnAP-ATM-KO-CL.1** | 0.003 | >10 |

The HSA (Highest Single Agent) Synergy Score was calculated according to Bernenbaum 1989 when the AZD5305 and enzalutamide were used in combination.

| **Cell Line** | **Synergy score** | |
|---|---|---|
| | **HSA** | **±SD** |
| **LnCAP parental** | 0.49 | 0.47 |
| **LnAP-ATM-KO-CL.1** | 5.5 | 1.1 |
| **LnAP-ATM-KO-CL.2** | 4.9 | 1.1 |

A stronger synergistic effect was seen in the LNCAP-ATM-KO models, compared to the LNCAP control cell line.

### Example 3. Efficacy of AZD5305 combined with enzalutamide in an in vivo pre-clinical model LNCaP

LNCaP cells (1x10⁷ cells 1:1 in Matrigel) were implanted subcutaneously onto the flank of male NOD SCID mice (aged 5-8 weeks weighing approximately 25-30 g, supplied by Charles River) using a 23-gauge needle. When tumours reached approximately 150 mm³, 40 mice with the most similar sized tumours were randomly assigned to treatment groups as demonstrated in the table below.

| **Group** | **n** | **Treatment** | **Dose** | **Schedule** |
|---|---|---|---|---|
| **1** | 10 | Vehicle | | |
| **2** | 10 | Enzalutamide | 4 mg/kg | QD |
| **3** | 10 | AZD5305 | 1 mg/kg | QD |
| **4** | 10 | Enzalutamide^{&} + AZD5305 | 4 mg/kg + 1 mg/kg | QD + QD |

| | | | | |
|---|---|---|---|---|
| & - Enzalutamide was given 1h prior to the AZD5305 morning dose | | | | |

### Dosing formulations

| | **Formulation** | **Concentration** |
|---|---|---|
| **Enzalutamide** | 5% DMSO: 95% Methylcellulose (0.5% w/v) with 0.1% Tween-80 | 0.4 mg/ml |
| **AZD5305** | sterile deionized water/HCl pH 3.5-4 | 0.1 mg/ml |

### Study

The mice were dosed for 42 days, with the dose calculated for individual animals on day of dosing, and with a 10mg/kg dosing volume.

### Tumour measurement

Tumours were measured x3 per week using digital calipers. The length and width of the tumour will be measured and volume calculated using the following formula: $volume = \left(length\times {width}^{2}\right) / 2 .$

### Bodyweight

The bodyweight of all mice in the study was measured and recorded 3 times per week; this information was used to calculate precise dosing for each animal.

### Results

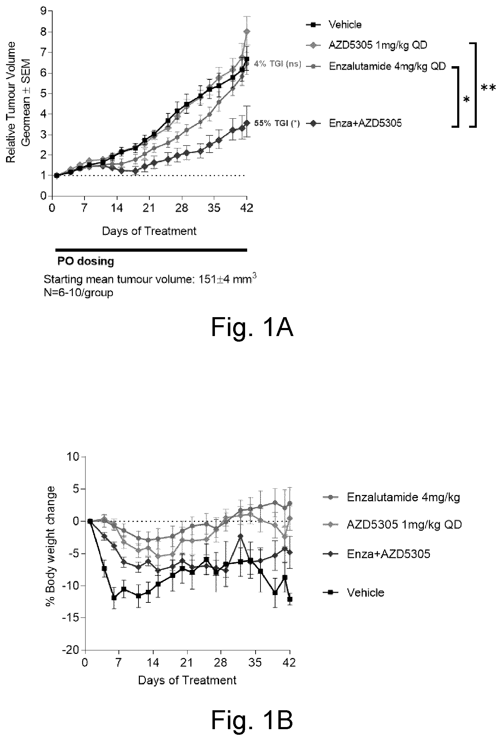

LnCAP tumours were insensitive (See Fig. 1A) to either enzalutamide 4 mg/kg or AZD5305 1 mg/kg once daily dosing (QD) and these agents as monotherapy caused no or minimal (4%) tumour growth inhibition (TGI). However, when combined, AZD5305 + enzalutamide caused significant 55% TGI vs control vehicle treated group. Moreover, this effect was significantly better than the effects observed in each monotherapy group. Statistical significance was evaluated compared with the vehicle or combination group using a one-tailed t test (*, P ≤ 0.05; **, P ≤ 0.01; ***, P ≤ 0.001; ns, not significant P > 0.05).

Both monotherapies and combination treatment were well tolerated (See Fig. 1B), and treated mice showed minimal body weight changes.

### Example 4. Efficacy of AZD5305 combined with enzalutamide in an in vivo pre-clinical model C901

C901 patient derived xenograft (PDX) model was engrafted subcutaneously onto the flank of donor mice and monitored once a week until ethical size (1000-2000 mm³). When donor tumours reached ethical size, PDX fragments measuring approximately 20 mm³ were engrafted in the study mice (male NMRI Nude mice, aged 6-9 weeks, supplied by Janvier labs). When tumours reached approximately 150 mm³, 40 mice with the most similar sized tumours were randomly assigned to treatment groups as demonstrated in the table below.

| **Group** | **n** | **Treatment** | **Dose** | **Schedule** |
|---|---|---|---|---|
| **1** | 8 | Vehicle | | |
| **2** | 8 | Enzalutamide | 60 mg/kg | QD |
| **3** | 8 | AZD5305 | 1 mg/kg | QD |
| **4** | 8 | Enzalutamide^{&} + AZD5305 | 60 mg/kg + 1 mg/kg | QD + QD |

| | | | | |
|---|---|---|---|---|
| & - Enzalutamide was given 1h prior to the AZD5305 morning dose | | | | |

### Dosing formulations

| | **Formulation** | **Concentration** |
|---|---|---|
| **Enzalutamide** | 5% DMSO: 95% Methylcellulose (0.5% w/v) with 0.1% Tween-80 | 6 mg/ml |
| **AZD5305** | sterile deionized water/HCl pH 3.5-4 | 0.1 mg/ml |

### Study

The mice were dosed for 21 days, with the dose calculated for individual animals on day of dosing, and with a 10mg/kg dosing volume.

### Tumour measurement

Tumours were measured x2 per week using digital calipers. The length and width of the tumour will be measured and volume calculated using the following formula: $volume = \left(length\times {width}^{2}\right) \times π / 6 .$

### Bodyweight

The bodyweight of all mice in the study was measured and recorded 2 times per week; this information was used to calculate precise dosing for each animal.

### Results

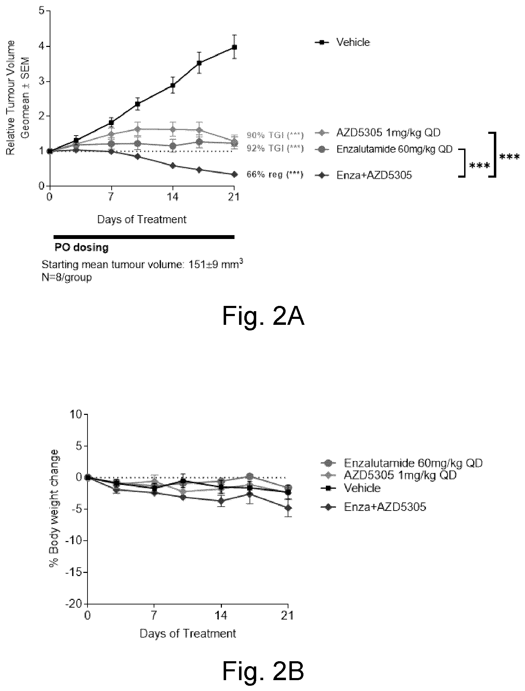

C901 tumours were sensitive to both enzalutamide 60 mg/kg or AZD5305 1 mg/kg once daily dosing (QD) and these agents as monotherapy caused significant TGI (92% and 90%, respectively) compared to control vehicle group (see Fig. 2A). Moreover, when combined, AZD5305 + enzalutamide caused further inhibition of tumour growth, resulting in 66% regression (reg) vs control vehicle treated group. Moreover, this effect was significantly better than the effects observed in each monotherapy group. Statistical significance was evaluated compared with the vehicle or combination group using a one-tailed t test (*, P ≤ 0.05; **, P ≤ 0.01; ***, P ≤ 0.001; ns).

Both monotherapies and combination treatment were well tolerated, and treated mice showed minimal body weight changes (See Fig. 2B).

### Example 5. Clinical Study of combination of AZD5305 and enzalutamide to treat mCRPC and mHSPC

### Inclusion Criteria

- Patients must have a histologically confirmed diagnosis of metastatic prostate cancer.
- Candidate for treatment with enzalutamide with documented current evidence of metastatic prostate cancer, where metastatic status is defined as at least one documented metastatic lesion on either bone scan or CT/MRI scan.
- Surgically or medically castrated, with serum testosterone levels ≤ 50 ng/dL (≤ 1.75 nmol/L) within (≤) 28 days before first dose of study treatment. Ongoing ADT with a GnRH agonist or antagonist for patients who have not undergone bilateral orchiectomy must be initiated at least 2 weeks before enrolment and must continue throughout the study.
- Patients must have either:
   (a) Metastatic Castrate Resistant Prostate Cancer.

Patients with mCRPC should have documented prostate cancer progression at screening as assessed by the Investigator with at least one of the following:
(i) PSA (prostate-specific antigen) progression defined by a minimum of 3 rising PSA levels with an interval of ≥ 1 week between each determination. The PSA value at the screening visit should be ≥ 1 µg/L (1 ng/mL).
(ii) Radiographic progression of soft tissue disease by RECIST criteria with or without PSA progression.
(iii) Radiographic progression of bone metastasis with two or more documented new bone lesions on a bone scan with or without PSA progression.

Patients with mCRPC should be either first or second line in the castrate resistant setting (should have received ≤ 1 prior line of systemic therapy). Androgen deprivation therapy does not count as a line of therapy. Docetaxel that was previously used when the patient was in the hormone sensitive stage of their disease would not count as a line of therapy.
OR

(b) Metastatic Hormone Sensitive Prostate Cancer.

For patients with mHSPC, the following prior therapies are permitted:
(i) Prior treatment with oestrogens, cyproterone acetate, or first-generation antiandrogens are permitted so long as treatment is discontinued 3 weeks or 5 half-lives (whichever is shorter) prior to enrolment.
(ii) ≤ 6 months of ADT prior to enrolment is permitted. Androgen deprivation therapy treatment should continue on study.
(iii) Patients may have received disease-related radiation or surgery; which should have been completed at least 4 weeks prior to enrolment.
   - Adequate organ and marrow function (in the absence of transfusions or growth factor support within 14 days prior to enrolment) as defined below :

| **Category** | **Parameter** | **Value** |
|---|---|---|
| **Haematological** | Haemoglobin | ≥ 10.0 g/dL |
| | Absolute neutrophil count | ≥ 1.5 × 10⁹/L |
| | Platelet count | ≥ 100 × 10⁹/L |
| **Hepatic** | Total bilirubin | ≤ 1.5 × ULN; ≤ 3 × ULN if the patient has Gilbert's syndrome |
| | ALT and AST | ≤ 2.5 × ULN in the absence of liver metastases |
| | | ≤ 5 × ULN^{a} in presence of liver metastases |
| | Albumin | ≥ 3 g/dL |
| | INR | ≤ 1.5 Patient receiving non-Vitamin K antagonist oral anticoagulants may be enrolled with an INR of < 2 |
| **Renal** | Calculated creatinine clearance by Cockcroft-Gault | ≥ 45 mL/minute |

| | | |
|---|---|---|
| ^{a} In the presence of liver metastases and raised ALT/AST between 2.5-5 × ULN, patients can only be enrolled if total bilirubin level is < 1.5 × ULN. ALT = alanine transaminase; AST = aspartate transaminase; INR = international normalized ratio; ULN = upper limit normal. | | |

- ECOG PS (Eastern Cooperative Oncology Group Performance Status): 0-1 with no deterioration over the previous 2 weeks.
- Life expectancy ≥ 16 weeks.

### Enzalutamide and AZD5305 Dose Escalation

The starting dose of AZD5305 will be 60mg once daily (QD). Enzalutamide will be dosed at 160 mg once daily (QD) with concurrent dosing of AZD5305 in combination with enzalutamide from day 1 of cycle 1, which will follow an initial cycle 0 of AZD5305 60 mg once daily (OD) dosed as a run-in for 7-days.

In the study, the cycle length will be 28 days, with AZD5305 being dosed once daily, and enzalutamide being dosed once daily at 160 mg. AZD5305 and enzalutamide will be taken on an empty stomach with no food for 2 hours and 1 hour after. The 160mg dose of enzalutamide will be taken as four 40mg soft capsule.

If the starting dose of AZD5305 of 60 mg QD is tolerated, the dose may be escalated to 90 mg QD if required (whilst the enzalutamide dose will be maintained at 160 mg QD), and if not tolerated, the AZD5305 dose will be de-escalated to 40 mg QD.

The dose of AZD5305 may be further escalated, up to no more than 140 mg QD.

The dose of AZD5305 may be de-escalated to 20 mg QD, either due to tolerability or if such dose is shown to be effective.

All potential dose escalation and/or de-escalation levels (including intermediate dose levels and exploration of alternative schedules of AZD5305) after the starting dose may be adjusted in light of emerging safety, tolerability and/or PK data.

### References

A number of publications are cited above in order to more fully describe and disclose the invention and the state of the art to which the invention pertains. Full citations for these references are provided below.

| | **Reference** | **Doi** |
|---|---|---|
| Armstrong 2019 | Armstrong AJ, et al., J Clin Oncol. 2019 Nov 10; 37(32): 2974-2986 | 10.1200/JC0.2019.37.7_suppl.687 |
| Asim 2017 | Asim M, et al., Nat Commun. 2017 Aug 29;8(1):374 | 10.1038/s41467-017-00393-y |
| Beer 2014 | Beer TM, et al., N Engl J Med. 2014 July 31; 371(5):424-433 | 10.1056/NEJMoa1405095 |
| Berenbaum 1989 | Berenbaum MC, Pharmacol Rev, 41 (1989), pp. 93-141 | PMID: 2692037 |
| Chappuis and Foulkes 2002 | Chappuis PO and Foulkes WD, Cancer Treat Res, 107, 29-59 (2002) | 10.1007/978-1-4757-3587-1_2 |
| Clarke 2018 | Clarke N, et al. Lancet Oncol 2018; 19: 975-986 | 10.1016/S1470-2045(18)30365-6 |
| Cunningham and You 2015 | Cunningham D and You Z., J Biol Methods 2015;2(1):e17 | 10.14440/jbm.2015.63 |
| Davies 2012 | Davies BR, et al., Mol Cancer Ther (2012) 11(4): 873-887 | 10.1158/1535-7163.MCT-11-0824-T |
| Dirix 2016 | Dirix L, et al., Clin Therapeutics (2016) 38(10): 2286-2299 | 10.1016/j.clinthera.2016.08.010 |
| Fizazi 2015 | Fizazi K, et al. Expert Rev Anticancer Ther. 2015 15(9):1007-1017 | 10.1586/14737140.2015.1081566 |
| Fizazi 2019 | Fizazi K, et al., N Engl J Med. 2019 Mar 28; 380(13):1235-1246 | 10.1056/NEJMoa1815671 |
| Gibbons 2015 | Gibbons J, et al., Clin Pharmacokinet (2015) 54:1057-1069 | 10.1007/s40262-015-0283-1 |
| Goodwin 2013 | Goodwin JF, et al., Cancer Discov. 3, 1254-1271 (2013) | 10.1158/2159-8290.CD-13-0108 |
| Hughes-Davies 2003 | Hughes-Davies, et al., Cell, 115, 523-535 (2003) | 10.1016/s0092-8674(03)00930-9 |
| Janatová 2003 | Janatová M, et al., Neoplasma, 50(4), 246-50 (2003) | PMID: 12937835 |
| Jancarkova 2003 | Jancárková N, Ceska Gynekol., 68(1), 11-6 (2003) | PMID: 12708108 |
| Jasmin 2002 | Jasin M, Oncogene, 21(58), 8981-93 (2002) | 10.1038/sj.onc.1206176 |
| Johannes 2021 | Johannes JW, et al., J Med Chem 2021, 64, 14498-14512 | 10.1021/acs.jmedchem.1c01012 |
| Khanna and Jackson 2001 | Khanna KK and Jackson SP, Nat. Genet. 27(3): 247-254 (2001) | 10.1038/85798 |
| Li 2017 | Li L, et al. Sci Signal. 2017 May 23;10(480):eaam7479 | 10.1126/scisignal.aam7479 |
| Moilanen 2015 | Moilanen A-M et al., Sci Rep. 2015 Jul 3; 5:12007 | 10.1038/srep12007 |
| Mohler 2019 | Mohler JL, et al., J Natl Compr Canc Netw. 2019 May 1; 17(5):479-505 | 10.6004/jnccn.2019.0023 |
| Neuhausen and Ostrander 1992 | Neuhausen SL and Ostrander EA, Genet. Test, 1, 75-83 (1992) | 10.1089/gte.1997.1.75 |
| Nyquist 2013 | Nyquist MD, et al., 2013 PNAS 110(43), 17492-17497 | 10.1073/pnas.1308587110 |
| Parker 2020 | Parker C, et al., Ann Oncol. 2020 Sep;31(9):1119-1134 | 10.1016/j.annonc.2020.06.011 |
| Polkinghorn 2013 | Polkinghorn WR, et al., Cancer Discov. 2013 Nov;3(11):1245-53 | 10.1158/2159-8290.CD-13-0172 |
| Potter 1995 | Potter GA, et al., J. Med. Chem. 1995, 38, 13, 2463-2471 | 10.1021/jm00013a022 |
| Ryan 2013 | Ryan CJ, et al., N Engl J Med. 2013 Jan 10; 368(2):138-148 | 10.1056/NEJMoa1209096 |
| Scher 2015 | Scher HI, et al., PLoS One. 2015 Oct 13; 10(10):e039440 | 10.1371/journal.pone.0139440 |
| Schiewer 2012 | Schiewer MJ, et al., Cancer Discov. 2012 Dec; 2(12):1134-1149 | 10.1158/2159-8290.CD-12-0120 |
| Schiewer and Knudsen 2014 | Schiewer MJ and Knudsen KE, Mol Cancer Res. 2014 Aug;12(8):1069-1080 | 10.1158/1541-7786.MCR-13-0672 |
| Shore 2019 | Shore N, et al., Targeted Oncology (2019) 14:527-539 | 10.1007/s11523-019-00674-0 |
| Sung 2021 | Sung H, et al., CA Cancer J Clin. 2021 May; 71(3):209-249 | 10.3322/caac.21660 |
| Tarish 2015 | Tarish FL, et al., Sci Transl Med. 2015 Nov 4;7(312):312re11 | 10.1126/scitranslmed.aac5671 |
| Tutt 2002 | Tutt, et al., Trends Mol Med., 8(12), 571-576, (2002) | 10.1016/s1471-4914(02)02434-6 |
| Wood 2001 | Wood, et al., Science, 291, 1284-1289 (2001) | 10.1126/science.1056154 |

## Claims

1. AZD5305, or a pharmaceutically acceptable salt thereof, for use in the treatment of metastatic prostate cancer, hormone sensitive prostate cancer (HSPC) or castrate resistant prostate cancer (CRPC) in a subject, wherein said treatment comprises the separate, sequential or simultaneous administration of i) said AZD5305, or a pharmaceutically acceptable salt thereof, and ii) enzalutamide, or a pharmaceutically acceptable salt thereof, to said subject.

2. AZD5305, or a pharmaceutically acceptable salt thereof, for use according to claim 1, wherein the metastatic prostate cancer is metastatic hormone sensitive prostate cancer (mHSPC) or metastatic castrate resistant prostate cancer (mCRPC).

3. AZD5305, or a pharmaceutically acceptable salt thereof, for use according to either claim 1 or claim 2, wherein AZD5305 is administered once daily.

4. AZD5305, or a pharmaceutically acceptable salt thereof, for use according to claim 3, wherein AZD5305 is administered in a dose of up to about 60 mg per day.

5. AZD5305, or a pharmaceutically acceptable salt thereof, for use according to claim 4, wherein AZD5305 is administered in a dose of 60 mg per day.

6. AZD5305, or a pharmaceutically acceptable salt thereof, for use according to any one of claims 1 to 5, wherein enzalutamide or a pharmaceutically acceptable salt thereof is administered once daily.

7. AZD5305, or a pharmaceutically acceptable salt thereof, for use according to claim 6, wherein enzalutamide or a pharmaceutically acceptable salt thereof is administered in a dose of 160 mg once daily.

8. AZD5305, or a pharmaceutically acceptable salt thereof, for use according to any one of claims 1 to 7, wherein AZD5305 and enzalutamide are taken together on an empty stomach, with no food two hours before.

9. Enzalutamide, or a pharmaceutically acceptable salt thereof, for use in the treatment of metastatic prostate cancer, hormone sensitive prostate cancer (HSPC) or castrate resistant prostate cancer (CRPC) in a subject, wherein said treatment comprises the separate, sequential or simultaneous administration of i) said enzalutamide, or a pharmaceutically acceptable salt thereof, and ii) AZD5305, or a pharmaceutically acceptable salt thereof, to said subject.

10. Enzalutamide, or a pharmaceutically acceptable salt thereof, for use according to claim 9, wherein the metastatic prostate cancer is metastatic hormone sensitive prostate cancer (mHSPC) or metastatic castrate resistant prostate cancer (mCRPC).

11. Enzalutamide, or a pharmaceutically acceptable salt thereof, for use according to either claim 9 or claim 10, wherein AZD5305 is administered once daily.

12. Enzalutamide, or a pharmaceutically acceptable salt thereof, for use according to claim 11, wherein AZD5305 is administered in a dose of up to about 60 mg per day.

13. Enzalutamide, or a pharmaceutically acceptable salt thereof, for use according to claim 12, wherein AZD5305 is administered in a dose of 60 mg per day.

14. A pharmaceutical product comprising i) AZD5305 or a pharmaceutically acceptable salt thereof, and ii) enzalutamide or a pharmaceutically acceptable salt thereof.

15. A kit comprising: a first pharmaceutical composition comprising AZD5305, or a pharmaceutically acceptable salt thereof; a second pharmaceutical composition comprising enzalutamide, or a pharmaceutically acceptable salt thereof; and instructions for using the first and second pharmaceutical compositions in combination.

## Patentansprüche

1. AZD5305, oder ein pharmazeutisch verträgliches Salz davon, zur Verwendung in der Behandlung von metastasenbildendem Prostatakrebs, hormonsensitivem Prostatakrebs (HSPC) oder kastrationsresistentem Prostatakrebs (CRPC) in einem Subjekt, wobei die Behandlung die getrennte, aufeinanderfolgende oder gleichzeitige Verabreichung von i) dem AZD5305, oder einem pharmazeutisch verträglichen Salz davon, und ii) Enzalutamid, oder einem pharmazeutisch verträglichen Salz davon, an das Subjekt umfasst.

2. AZD5305, oder ein pharmazeutisch verträgliches Salz davon, zur Verwendung nach Anspruch 1, wobei der metastasenbildende Prostatakrebs metastasenbildender hormonsensitiver Prostatakrebs (mHSPC) oder metastasenbildender kastrationsresistenter Prostatakrebs (mCRPC) ist.

3. AZD5305, oder ein pharmazeutisch verträgliches Salz davon, zur Verwendung nach Anspruch 1 oder 2, wobei AZD5305 einmal täglich verabreicht wird.

4. AZD5305, oder ein pharmazeutisch verträgliches Salz davon, zur Verwendung nach Anspruch 3, wobei AZD5305 in einer Dosis von bis zu etwa 60 mg pro Tag verabreicht wird.

5. AZD5305, oder ein pharmazeutisch verträgliches Salz davon, zur Verwendung nach Anspruch 4, wobei AZD5305 in einer Dosis von 60 mg pro Tag verabreicht wird.

6. AZD5305, oder ein pharmazeutisch verträgliches Salz davon, zur Verwendung nach einem der Ansprüche 1 bis 5, wobei Enzalutamid oder ein pharmazeutisch verträgliches Salz davon einmal täglich verabreicht wird.

7. AZD5305, oder ein pharmazeutisch verträgliches Salz davon, zur Verwendung nach Anspruch 6, wobei Enzalutamid oder ein pharmazeutisch verträgliches Salz davon in einer Dosis von 160 mg einmal täglich verabreicht wird.

8. AZD5305, oder ein pharmazeutisch verträgliches Salz davon, zur Verwendung nach einem der Ansprüche 1 bis 7, wobei AZD5305 und Enzalutamid zusammen auf nüchternen Magen ohne Nahrung zwei Stunden zuvor eingenommen werden.

9. Enzalutamid, oder ein pharmazeutisch verträgliches Salz davon, zur Verwendung in der Behandlung von metastasenbildendem Prostatakrebs, hormonsensitivem Prostatakrebs (HSPC) oder kastrationsresistentem Prostatakrebs (CRPC) in einem Subjekt, wobei die Behandlung die getrennte, aufeinanderfolgende oder gleichzeitige Verabreichung von i) dem Enzalutamid, oder einem pharmazeutisch verträglichen Salz davon, und ii) AZD5305, oder einem pharmazeutisch verträglichen Salz davon, an das Subjekt umfasst.

10. Enzalutamid, oder ein pharmazeutisch verträgliches Salz davon, zur Verwendung nach Anspruch 9, wobei der metastasenbildende Prostatakrebs metastasenbildender hormonsensitiver Prostatakrebs (mHSPC) oder metastasenbildender kastrationsresistenter Prostatakrebs (mCRPC) ist.

11. Enzalutamid, oder ein pharmazeutisch verträgliches Salz davon, zur Verwendung nach Anspruch 9 oder 10, wobei AZD5305 einmal täglich verabreicht wird.

12. Enzalutamid, oder ein pharmazeutisch verträgliches Salz davon, zur Verwendung nach Anspruch 11, wobei AZD5305 in einer Dosis von bis zu etwa 60 mg pro Tag verabreicht wird.

13. Enzalutamid, oder ein pharmazeutisch verträgliches Salz davon, zur Verwendung nach Anspruch 12, wobei AZD5305 in einer Dosis von 60 mg pro Tag verabreicht wird.

14. Pharmazeutisches Produkt, umfassend i) AZD5305 oder ein pharmazeutisch verträgliches Salz davon, und ii) Enzalutamid oder ein pharmazeutisch verträgliches Salz davon.

15. Kit, umfassend: eine erste pharmazeutische Zusammensetzung, umfassend AZD5305, oder ein pharmazeutisch verträgliches Salz davon; eine zweite pharmazeutische Zusammensetzung, umfassend Enzalutamid, oder ein pharmazeutisch verträgliches Salz davon; und Anweisungen zum Verwenden der ersten und der zweiten pharmazeutischen Zusammensetzung in Kombination.

## Revendications

1. AZD5305, ou sel pharmaceutiquement acceptable de celui-ci, pour une utilisation dans le traitement du cancer de la prostate métastatique, du cancer de la prostate hormonosensible (HSPC) ou du cancer de la prostate résistant à la castration (CPRC) chez un sujet, dans lequel ledit traitement comprend l'administration séparée, séquentielle ou simultanée i) dudit AZD5305, ou d'un sel pharmaceutiquement acceptable de celui-ci, et ii) d'enzalutamide, ou d'un sel pharmaceutiquement acceptable de celui-ci, audit sujet.

2. AZD5305, ou sel pharmaceutiquement acceptable de celui-ci, pour une utilisation selon la revendication 1, dans lequel le cancer de la prostate métastatique est un cancer de la prostate métastatique hormonosensible (mHSPC) ou un cancer de la prostate métastatique résistant à la castration (mCPRC).

3. AZD5305, ou sel pharmaceutiquement acceptable de celui-ci, pour une utilisation selon la revendication 1 ou la revendication 2, dans lequel l'AZD5305 est administré une fois par jour.

4. AZD5305, ou sel pharmaceutiquement acceptable de celui-ci, pour une utilisation selon la revendication 3, dans lequel l'AZD5305 est administré à une dose allant jusqu'à environ 60 mg par jour.

5. AZD5305, ou sel pharmaceutiquement acceptable de celui-ci, pour une utilisation selon la revendication 4, dans lequel l'AZD5305 est administré à une dose de 60 mg par jour.

6. AZD5305, ou sel pharmaceutiquement acceptable de celui-ci, pour une utilisation selon l'une quelconque des revendications 1 à 5, dans lequel l'enzalutamide ou un sel pharmaceutiquement acceptable de celui-ci est administré une fois par jour.

7. AZD5305, ou sel pharmaceutiquement acceptable de celui-ci, pour une utilisation selon la revendication 6, dans lequel l'enzalutamide ou un sel pharmaceutiquement acceptable de celui-ci est administré à une dose de 160 mg une fois par jour.

8. AZD5305, ou sel pharmaceutiquement acceptable de celui-ci, pour une utilisation selon l'une quelconque des revendications 1 à 7, dans lequel l'AZD5305 et l'enzalutamide sont pris ensemble à jeun, sans nourriture deux heures avant.

9. Enzalutamide, ou sel pharmaceutiquement acceptable de celui-ci, pour une utilisation dans le traitement du cancer de la prostate métastatique, du cancer de la prostate hormonosensible (HSPC) ou du cancer de la prostate résistant à la castration (CPRC) chez un sujet, dans lequel ledit traitement comprend l'administration séparée, séquentielle ou simultanée i) dudit enzalutamide, ou d'un sel pharmaceutiquement acceptable de celui-ci, et ii) d'AZD5305, ou d'un sel pharmaceutiquement acceptable de celui-ci, audit sujet.

10. Enzalutamide, ou sel pharmaceutiquement acceptable de celui-ci, pour une utilisation selon la revendication 9, dans lequel le cancer de la prostate métastatique est un cancer de la prostate métastatique hormonosensible (mHSPC) ou un cancer de la prostate métastatique résistant à la castration (mCPRC).

11. Enzalutamide, ou sel pharmaceutiquement acceptable de celui-ci, pour une utilisation selon la revendication 9 ou la revendication 10, dans lequel l'AZD5305 est administré une fois par jour.

12. Enzalutamide, ou sel pharmaceutiquement acceptable de celui-ci, pour une utilisation selon la revendication 11, dans lequel l'AZD5305 est administré à une dose allant jusqu'à environ 60 mg par jour.

13. Enzalutamide, ou sel pharmaceutiquement acceptable de celui-ci, pour une utilisation selon la revendication 12, dans lequel l'AZD5305 est administré à une dose de 60 mg par jour.

14. Produit pharmaceutique comprenant i) de l'AZD5305 ou un sel pharmaceutiquement acceptable de celui-ci, et ii) de l'enzalutamide ou un sel pharmaceutiquement acceptable de celui-ci.

15. Kit comprenant : une première composition pharmaceutique comprenant de l'AZD5305, ou un sel pharmaceutiquement acceptable de celui-ci ; une seconde composition pharmaceutique comprenant de l'enzalutamide, ou un sel pharmaceutiquement acceptable de celui-ci ; et des instructions pour l'utilisation des première et seconde compositions pharmaceutiques en combinaison.
